# EUROPÄISCHE PATENTANMELDUNG

(11) **EP 3 760 411 A1**
(43) Veröffentlichungstag der Anmeldung: **06.01.2021**
(21) Anmeldenummer: 19183857.2
(22) Anmeldetag: 02.07.2019
(51) Int. Cl.: B29C 49/00, B29B 15/00, C08J 7/02, C08J 7/06, A61M 25/00, A61M 25/10, B29K 77/00

(54) **VERFAHREN ZUM MODIFIZIEREN EINER OBERFLÄCHE EINES BALLONS FÜR EINEN BALLONKATHETER**

(71) Anmelder: BIOTRONIK AG, 8180 Bülach (CH)
(72) Erfinder: Quint, Bodo, 79802 Dettighofen (DE)
(74) Vertreter: Randoll, Sören

(57) **Zusammenfassung**

Die Erfindung betrifft ein Verfahren zur Herstellung eines Ballons (1) für einen Ballonkatheter (2), aufweisend die Schritte: Bereitstellen eines schlauchförmigen Vorformlings (10), der eine ein erstes Polymer (11) aufweisende äußere Oberfläche (10a) aufweist, Kontaktieren der äußeren Oberfläche (10a) mit einer Substanz (13) aufweisend ein Lösungsmittel, so dass das erste Polymer (11) an der äußeren Oberfläche (10a) angelöst wird, und Blasformen des Vorformlings (10) unter Erzeugung des Ballons (1). Weiterhin betrifft die Erfindung einen durch das Verfahren hergestellten Ballon bzw. Ballonkatheter sowie ferner eine Verwendung.

## Beschreibung

Ballons für Ballonkatheter können z.B. durch Blasformen eines Ballonschlauchs (Vorformling) hergestellt werden. Hierbei wurde festgestellt, dass die maximal erreichbare Deformation eines Ballonschlauches nicht durch die Dimensionierung an der Innenseite des Ballonschlauches begrenzt ist, sondern dass die äußere Dimensionierung des Ballonschlauches begrenzend wirkt. Bedingt durch die Herstellung des Ballonschlauches aus einem Polymer mittels Extrusion ist das Polymer des Ballonschlauches an der Außenseite des Ballonschlauchs stark orientiert, was z.B. mittels Beugungsuntersuchungen und Infrarotspektroskopie gut nachweisbar ist. Des Weiteren ist bekannt, dass mit zunehmender Orientierung des Polymers z.B. die Festigkeit von Polyamiden gesteigert werden kann.

Aus dem Stand der Technik ist es bekannt, Ballonschläuche mittels Co-Extrusion mit einem inwandig festerem und einem außenwandig reibungsintensiveren Material herzustellen.

Die Co-Extrusion erhöht dabei systematisch die Scherbedingungen in der Schmelze und führt somit verglichen mit einer ähnlich dimensionierten Schlauchextrusion zu gesteigerten Polymerorientierungen. Sehr dünne Co-Extrusionslagen (kleiner als 5 Mikrometer) sind äußerst schwer reproduzierbar. Sowohl der in der Co-Extrusion reduzierte Materialfluss, wie auch die gesteigerten Scherbedingungen können zu einer intensiveren thermisch oxidativer Vorschädigung des Polymermateriales führen. Diese Schädigung ist bei medizintechnisch spezifizierten Polyamiden intensiver als bei entsprechenden "technischen" Polymervarianten, da bei diesen Polyamiden in der Regel auf entsprechende Polymeradditive verzichtet wird.

Hiervon ausgehend liegt der vorliegenden Erfindung die Aufgabe zugrunde ein Verfahren zur Herstellung eines Ballons für einen Ballonkatheter anzugeben, dass eine verbesserte Modifizierung einer äußeren Oberfläche des Ballons ermöglicht.

Dieses Problem wird durch ein Verfahren mit den Merkmalen des Anspruchs 1, einen Ballon mit den Merkmalen des Anspruchs 10 sowie durch eine Verwendung mit den Merkmalen des Anspruchs 12 gelöst. Vorteilhafte Ausgestaltungen dieser Erfindungsaspekte sind in den entsprechenden Unteransprüchen angeben und werden nachfolgend beschrieben.

Demgemäß wird ein Verfahren zur Herstellung eines Ballons vorgeschlagen, insbesondere für einen Ballonkatheter, offenbart, aufweisend die Schritte:
- Bereitstellen eines schlauchförmigen Vorformlings, der eine ein erstes Polymer aufweisende oder durch das Polymer gebildete äußere Oberfläche aufweist,
- Kontaktieren der äußeren Oberfläche mit einer flüssigen Substanz aufweisend ein Lösungsmittel (insbesondere eine Säure), so dass das erste Polymer an der äußeren Oberfläche angelöst wird, und
- Blasformen des Vorformlings unter Erzeugung eines Ballons.

Das Polymersubstrat des Vorformlings bzw. Ballonschlauches ist nicht als isotropes Bauteil zu verstehen. Bedingt z.B. durch einen Extrusionsprozess unterliegt die äußerste Randschicht bzw. die besagte äußere Oberfläche einer sehr stark erhöhten Polymerorientierung. Durch den kontrollierten Lösungsprozess wird die äußere Oberfläche angelöst und somit diese Polymerorientierung zumindest zum Teil aufgehoben. Dies wird vorzugsweise durch das Anlösen der Polymeroberfläche mit einem intensiven Lösungsmittel erreicht.

Hierdurch wird mit Vorteil die maximal erreichbare Expansionsrate des Ballons erhöht bzw. eine erhöhte Druckfestigkeit des Ballons bei gegebener Wandstärke erreicht. Praktische Designgrenzen, denen bisherige Ballondesigns unterlagen, können somit so erweitert werden, dass dünnwandigere und druckfestere Ballons hergestellt werden können.

Vorzugsweise ist bei dem erfindungsgemäßen Verfahren vorgesehen, dass während einer thermischen Fixierung des Ballons (bei z.B. 130°C -160°C) im Blasformprozess der Lösungsmittelanteil nahezu restlos ausgetrieben wird.

Weiterhin ist gemäß einer bevorzugten Ausführungsform des Verfahrens vorgesehen, dass das Lösungsmittel eine der folgenden Säuren ist: Trifluoressigsäure (C₂HF₃O₂, CAS-Nummer 76-05-1).

Gemäß einer Ausführungform des Verfahrens ist vorgesehen, dass die Substanz durch das Lösungsmittel gebildet ist, was insbesondere bedeutet, dass die Substanz z.B. keine weiteren Bestandteile wie z.B. ein Polymer in Lösung aufweist.

Gemäß einer alternativen Ausführungsform der Erfindung soll der Anlöseprozess so gestaltet sein, dass die Oberfläche bzw. Grenzschicht auch noch weiteren Eigenschaftsmodifikationen unterliegt. So kann z.B. neben dem reinen Anlöseprozess auch die kombinierte Polymerabscheidung an der Schlauchoberfläche vorgenommen werden. D.h. die besagte Substanz weist weiterhin ein gelöstes zweites Polymer auf, das auf der Oberfläche des Vorformlings unter Verdampfung des Lösungsmittels abgeschieden wird.

Hierbei kann z.B. ein rigides Basisballonmaterial (erstes Polymer) an der Oberfläche mit einem weicheren und reibungsintensiveren zweiten Polymer modifiziert werden.

Alternativ kann die Oberfläche mit einem weniger verformbaren bzw. härteren Polymer (z.B. ein aromatisches Polyamid) modifiziert werden, um einen druckfesteren Ballon bzw. Ballonbereich zu erzeugen.

Weiterhin kann durch die Wahl einer äußeren, weicheren Polyamidschicht bzw. Copolymerschicht (z.B. ein Polyetherblockamid (PEBA)) die sogenannte Stenthaltekraft, mit der ein Stent auf dem Ballon gehalten wird, gesteigert werden. Diese primär auf dem Reibungskoeffizienten beruhende Haftungssteigerung ist jedoch im Gegensatz zu einem auf Co-Extrusion beruhenden Design mit einer wesentlich dünner gestalteten Oberflächenschicht möglich.

Gemäß einer Ausführungsform des Verfahrens ist vorgesehen, dass das abgeschiedene zweite Polymer härter ist als das erste Polymer, oder dass das erste Polymer härter ist als das abgeschiedene zweite Polymer. Ein härteres Material kann beispielsweise in der Form vorliegen, dass es einen höheren Shore D Wert aufweist.

Weiterhin ist gemäß einer Ausführungsform des Verfahrens vorgesehen, dass das erste Polymer eines der folgenden Polymere ist: ein aliphatisches oder aromatisches Polyamid, oder ein thermoplastisches Polymer wie beispielsweise ein thermoplastisches elastomermodifiziertes Polymer wie z.B. ein Polyetherblockamid.

Weiterhin ist gemäß einer Ausführungsform des Verfahrens vorgesehen, dass das abgeschiedene zweite Polymer eines der folgenden Polymere ist: ein aliphatisches oder aromatisches Polyamid, oder ein thermoplastisches Polymer wie beispielsweise ein thermoplastisches elastomermodifiziertes Polymer wie z.B. ein Polyetherblockamid.

In einer Ausführungsform wird als zweites Polymer dasselbe Polymer aufgetragen wie das erste Polymer. Der Vorteil bei dieser Ausführungsform liegt darin, dass nur an der äußersten Ballonschicht eine vollkommen unorientierte Schicht erhalten wird. Ein solcher Ballon zeigt eine deutlich verringerte Rissbildung.

Die Verwendung von einem aromatischen Polyamids (Aramid) hat den besonderen Vorteil, dass diese Polymere extrem dünn aufgetragen werden können. Hierdurch können Ballone zur Verfügung gestellt werden, bei denen die Steifigkeit auf ein Minimum reduziert ist. Ferner zeigen solche Ballone eine vollkommen unorientierte Außenschicht, was zu einer stark verminderten Rissbildung führt.

Weiterhin ist gemäß einer Ausführungsform des Verfahrens vorgesehen, dass der Vorformling durch Extrusion aus einem Material hergestellt wird, dass das erste Polymer aufweist oder durch das erste Polymer gebildet ist.

Gemäß einem weiteren Aspekt der vorliegenden Erfindung wird ein Ballon für einen Ballonkatheter offenbart, wobei der Ballon mittels des erfindungsgemäßen Verfahrens hergestellt ist.

Ein weiterer Aspekt der vorliegenden Erfindung betrifft einen Ballonkatheter, der einen erfindungsgemäßen Ballon aufweist sowie vorzugsweise einen auf dem Ballon angeordneten Stent. Der Stent ist dabei vorzugsweise auf den gefalteten Ballon gecrimpt.

Ein weiterer Aspekt der vorliegenden Erfindung betrifft eine Verwendung von Trifluoressigsäure zum Anlösen einer Oberfläche, insbesondere einer Oberfläche eines schlauchförmigen Vorformlings, insbesondere eines Vorformlings für einen Ballon.

Hierbei ist die Oberfläche durch das erste Polymer gebildet oder weist dieses auf, wobei durch das Anlösen eine z.B. durch Extrusion des Vorformlings erzielte Orientierung des ersten Polymers an der Oberfläche herabgesetzt wird.

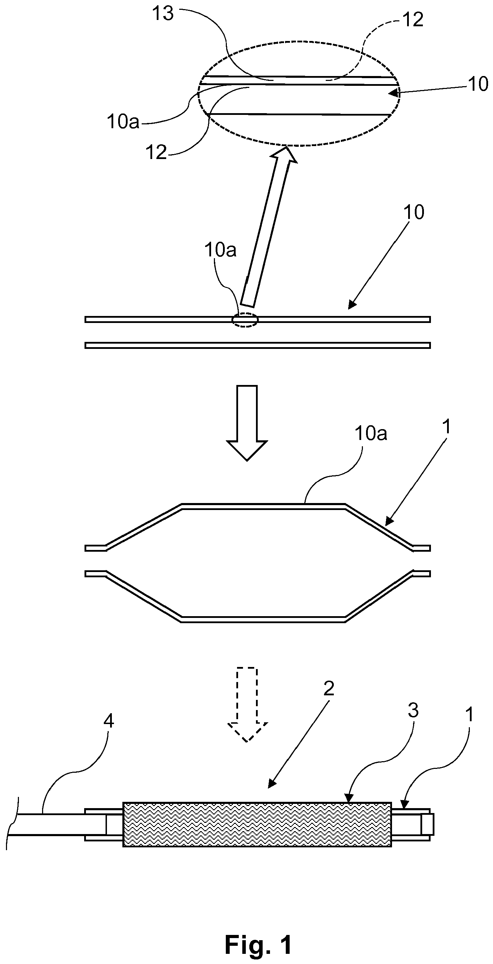

Im Folgenden sollen Ausführungsformen sowie weitere Merkmale und Vorteile der Erfindung anhand der Figur erläutert werden. Es zeigt
- Fig. 1: eine schematische Darstellung einer Ausführungsform des erfindungsgemäßen Verfahrens.

Fig. 1 zeigt eine schematische Darstellung einer Ausführungsform eines erfindungsgenmäßen Verfahrens zur Herstellung eines Ballons 1 für einen Ballonkatheter 2. Bei dem Verfahren wird ein schlauchförmiger Vorformling 10 bereitgestellt, der z.B. aus einem ersten Polymer 11, z.B. ein Polyamid, durch Extrusion erzeugt wird und entsprechend eine Oberfläche 10a mit einer vergleichsweise hohen Orientierung des ersten Polymers 11 an der Oberfläche 10a aufweist. Zur Reduzierung dieser Orientierung wird die äußere Oberfläche 10a vorzugsweise mit einem Lösungsmittel 13 bzw. einer lösemittelhaltigen Substanz 13 kontaktiert, so dass das erste Polymer 11 an der äußeren Oberfläche 10a angelöst wird. Hierdurch wird die Orientierung des ersten Polymers 11 herabgesetzt. In weiteren Schritten kann der Ballon 1 durch Blasformen des Vorformlings 10 erzeugt werden bzw. zu einem Katheter 2 komplettiert werden, wobei auf den gefalteten Ballon ein Stent 3 festgelegt werden kann. Der Katheter 2 weist einen Schaft 4 auf, wobei der Ballon am distalen Ende des Schafts 4 festgelegt wird.

Als Lösungsmittel wird vorzugweise Trifluoressigsäure verwendet. Gemäß einer Ausführungsform kann die Trifluoressigsäure lediglich zum Anlösen der Oberfläche 10a verwendet werden. Weiterhin kann jedoch zusätzlich ein zweites Polymer 12 aus der Lösung auf der Oberfläche 10a abgeschieden werden.

Hierbei erlaubt die Trifluoressigsäure mit Vorteil eine zeitlich schnelle Polymerabscheidung aus der Lösung.

Vorzugsweise wird zum Auftragen der Substanz 13 auf die äußere Oberfläche 10a des Vorformlings 10 ein diskontinuierliches Tauchverfahren verwendet. Dieses erlaubt insbesondere auch ein Applizieren der Substanz 13 an diskreten Stellen der Oberfläche 10a sowie des Weiteren einen kurzen Lösungsmittelkontakt wie auch eine geringe Ausziehgeschwindigkeit.

Diese langsame Ausziehgeschwindigkeit ermöglicht das kontrollierte Auftragen einer dünnen Schicht der Substanz 13, da für eine reproduzierbare Schichtdicke das Erreichen eines Gleichgewichtzustandes zwischen dem gravimetrisch und viskositätekontrollierten Rückfluss der Beschichtungslösung bzw. Substanz 13 und der Oberflächenspannung der Substanz 13 von Vorteil ist.

Ein Tauchprozess ist prinzipiell weniger zum Auftrag der Substanz 13 geeignet, da hier die axialen Bereiche des Vorformlings 10 unterschiedlich lange dem intensiven Lösungsmittelkontakt ausgesetzt sind. Gleichfalls erfordert ein Sprühprozess einen vergleichsweise großen Aufwand hinsichtlich der Sicherstellung einer reproduzierbaren Auftragsgeschwindigkeit und Auftragsmenge und stellt in der Regel auch eine weniger effektive Beschichtungsmethode dar.

Weiterhin ergibt sich hinsichtlich des Blasformens des Ballons 1 aus dem behandelten Vorformling 10 ein Synergieeffekt, da sich ein Restlösungsmittelgehalt in dem Vorformling bzw. Ballonschlauch 10 mit Vorteil wie ein Weichmacher innerhalb des Polymers verhält und somit zusätzlich Verformungskräfte wie auch -grenzen ändert.

Während der thermischen Fixierung (ca. 130-160°C) des Ballons 1 beim Blasformen wird dieser Lösungsmittelanteil vorzugsweise restlos ausgetrieben. Es ist technisch naheliegend das hierbei sowohl mit einer gesteigerten Kristallisation semikristalliner Polyamide gerechnet werden muss - was vorteilhaft deren Dimensionsstabilität und Druckfestigkeit (bei gleicher Prozesszeit) oder deren Produktivität (verringerte Prozesszeit) beeinflusst.

Durch die vorliegende Erfindung wird die Verformbarkeit des Vorformlings bzw. des Ballonschlauches 10 prinzipiell erhöht, so dass neue und erweiterte Designgrenzen der bisherigen Ballonschlauchauslegung ermöglicht werden.

Auf technisch einfach zu kontrollierendem Wege lassen sich sowohl druckfestere, wie auch vorteilhaft "reibungsintensivierte" Ballons für ballonbasierte Stent-Delivery-Systeme bzw. Ballonkatheter herstellen.

Insbesondere können druckfestere Ballons - verglichen zu entsprechenden Co-Extrudatdesigns - erhalten werden bzw. bei vergleichbarer Druckfestigkeit lassen sich dünnere Ballons realisieren. Insbesondere können mit Vorteil z.B. aromatische Polyamide (z.B. Aramid) auf der Ballonoberfläche abgeschieden werden, um besonders verstärkte Hochdruckballons zu erhalten.

## Patentansprüche

1. Verfahren zur Herstellung eines Ballons (1) für einen Ballonkatheter (2), aufweisend die Schritte:
- Bereitstellen eines schlauchförmigen Vorformlings (10), der eine ein erstes Polymer (11) aufweisende äußere Oberfläche (10a) aufweist,
- Kontaktieren der äußeren Oberfläche (10a) mit einer Substanz (13) aufweisend ein Lösungsmittel, so dass das erste Polymer (11) an der äußeren Oberfläche (10a) angelöst wird, und
- Blasformen des Vorformlings (10) unter Erzeugung des Ballons (1).

2. Verfahren nach Anspruch 1, wobei das Lösungsmittel eine der folgenden Säuren ist: Trifluoressigsäure.

3. Verfahren nach einem der vorhergehenden Ansprüche, wobei eine Orientierung des ersten Polymers (10) an der Oberfläche (10a) des Vorformlings (10) durch das Anlösen reduziert wird.

4. Verfahren nach einem der vorhergehenden Ansprüche, wobei die Substanz (13) durch das Lösungsmittel gebildet ist.

5. Verfahren nach einem der Ansprüche 1 bis 3, wobei die Substanz (13) weiterhin ein gelöstes zweites Polymer (12) enthält und das zweite Polymer (12) auf der Oberfläche (10a) des Vorformlings (10) unter Verdampfung des Lösungsmittels abgeschieden wird.

6. Verfahren nach einem der vorhergehenden Ansprüche, wobei das erste Polymer (11) eines der folgenden Polymere ist: ein aliphatisches oder aromatisches Polyamid, oder ein thermoplastisches elastomermodifiziertes Polymer wie z.B. ein Polyetherblockamid.

7. Verfahren nach Anspruch 5 oder nach Anspruch 6 sofern rückbezogen auf Anspruch 5, wobei das abgeschiedene zweite Polymer (12) eines der folgenden Polymere ist: ein aliphatisches oder aromatisches Polyamid, oder ein thermoplastisches Polymer.

8. Verfahren nach Anspruch 7, wobei das thermoplastische Polymer ein thermoplastisches elastomermodifiziertes Polymer ist.

9. Verfahren nach Anspruch 8, wobei thermoplastische elastomermodifizierte Polymer ein Polyetherblockamid ist

10. Verfahren nach Anspruch 5 oder einem der Ansprüche 7 bis 9, wobei das abgeschiedene zweite Polymer (12) härter ist als das erste Polymer (11), oder wobei das erste Polymer (11) härter ist als das abgeschiedene zweite Polymer (12).

11. Verfahren nach einem der vorhergehenden Ansprüche, wobei der Vorformling (10) durch Extrusion aus einem Material hergestellt wird, dass das erste Polymer (11) aufweist oder durch das erste Polymer (11) gebildet ist.

12. Ballon (1) für einen Ballonkatheter (2), wobei der Ballon mittels des Verfahrens nach einem der vorhergehenden Ansprüche hergestellt ist.

13. Ballonkatheter (2), aufweisend einen Ballon (1) nach Anspruch 12 sowie einen auf dem Ballon (1) angeordneten Stent (3).

14. Verwendung von Trifluoressigsäure zum Anlösen einer Oberfläche (10a) eines Vorformlings (10) für einen Ballon (1).
